# EUROPEAN PATENT SPECIFICATION

(11) **EP 0 759 088 B1**
(45) Date of publication and mention of the grant of the patent: **02.05.2001**
(21) Application number: 95910200.5
(22) Date of filing: 10.02.1995
(51) Int. Cl.: C12P 17/04

(54) **L-ASCORBIC ACID PRODUCTION IN MICROORGANISMS**
Herstellung von L - Ascorbinsäure in Mikroorganismen
PRODUCTION D'ACIDE L-ASCORBIQUE DANS DES MICROORGANISMES

(30) Priority: 10.02.1994 US 196338
(43) Date of publication of application: 26.02.1997
(73) Proprietor: DCV, Inc., Wilmington, Delaware 19810 (US)
(72) Inventor: HUSS, Ronald, John, Manitowoc, WI 54220 (US); RUNNING, Jeffrey, A., Manitowoc, WI 54220 (US); SKATRUD, Thomas, J., Manitowoc, WI 54220 (US)
(74) Representative: Griffin, Kenneth David
(86) International application number: US9501574
(87) International publication number: WO9521933

(56) References cited:
- EP-A- 0 207 763
- WO-A-93/19192
- WO-A-93/19193
- PLANT SCI. LETT. (1983), 28(3), 299-305 CODEN: PTSLAF;ISSN: 0304-4211, RENSTROEM, B. ET AL 'Biosynthesis of L- ascorbic acid in Chlorella pyrenoidosa'

## Description

### FIELD OF THE INVENTION

This invention relates to the production of L-ascorbic acid (Vitamin C) by microalgae. In particular, the invention relates to a method for the microalgal production of L-ascorbic acid at low pH (2.5-6.0).

### BACKGROUND OF THE INVENTION

L-Ascorbic acid (Vitamin C) is a water-soluble vitamin widely distributed in the plant and animal kingdom. L-Ascorbic acid (L-AA) can be extracted from plant sources, such as paprika, Gladiolus leaves, rose hips, persimmon, and citrus fruit, or synthesized from L-xylose, L-galactose, or D-glucose. F. A. Loewus, L-Ascorbic Acid: Metabolism, Biosynthesis, Function, in The Biochemistry of Plants, Vol. 3, Academic Press, New York, 1980, pp. 77-99, reviews the biosynthesis and sources of L-ascorbic acid.

Although most species of animals synthesize L-ascorbic acid, humans and other primates, guinea pigs, fruit eating bats, some birds, and fish such as Coho salmon, rainbow trout and carp cannot. These animals require a dietary source of L-ascorbic acid to prevent scurvy. L-ascorbic acid deficiency in fish also causes scoliosis, lordosis, reduced weight gain, increased susceptibility to bacterial infection, dark skin color, fin erosion, and reduced formation of bone cartilage.

L-Ascorbic acid is a bulk chemical that requires economic and efficient production methods. Various algae produce L-ascorbic acid. [See, for example, Aaronson, Arch. Microbiol., 112, 57-59 (1977) and Renstrom, Plant Sci. Letters, 28, 299-305 (1982/1983)]. Because carbon source utilization is poor and the acid is produced in low concentration, microalgal production of L-ascorbic acid has not been a useful large scale production method.

Skatrud, U.S. Patent 5,001,059, describes a method that uses microalgae of the species Chlorella pyrenoidosa to form L-ascorbic acid in high yield. Because L-ascorbic acid concentration and carbon source utilization were significantly improved, this method was a significant improvement in microalgal L-ascorbic acid production. However, at the relatively high pH necessary for continued production of L-ascorbic acid (6.5-7), extracellular acid is readily oxidized by the air required for cell metabolism and L-ascorbic acid production. A need exists for an efficient microalgal method for producing L-ascorbic acid at low pH (less than 6.5).

### SUMMARY OF THE INVENTION

The present invention is directed toward a process for the production of L-ascorbic acid. The process includes culturing an organism selected from the group consisting of organisms of the genus *Prototheca* and organisms of the species *Chlorella protothecoides* in a fermentation having a pH of less than about 6.0. The process further includes recovering L-ascorbic acid from the fermentation medium. An alternative embodiment includes a process for producing L-ascorbic acid by culturing an organism as identified above in a fermentation medium having an available source of oxygen, wherein the fermentation medium includes extracellular L-ascorbic acid and recovering L-ascorbic acid from the fermentation medium. Preferred organisms are organisms of the genus *Prototheca* and particularly preferred organisms are organisms of the species *Prototheca moriformis* and *Prototheca zopfii.* The process of the present invention is particularly advantageous because it can result in the production of significant concentrations of extracellular L-ascorbic acid which renders recovery of L-ascorbic acid simpler than if most L-ascorbic acid were to be segregated within cells. The present process can be conducted in the presence of an available source of oxygen because at the pH conditions of the present process, degradation of produced extracellular L-ascorbic acid is not significant.

Recovery of extracellular L-ascorbic acid can be achieved by a variety of processes, including ion exchange, chromatography, extraction, membrane separation, reverse osmosis, distillation, chemical derivatization processes and crystallization. The process can additionally include recovery of intracellular L-ascorbic acid. In one embodiment, cells are removed from the fermentation broth and extracellular L-ascorbic acid is recovered from the cell-free fermentation broth and L-ascorbic acid is separately recovered from the separated cells.

A further aspect of the present invention includes a fermentation culture which includes L-ascorbic acid-producing microalgae and a fermentation medium. In this embodiment, the fermentation medium includes extracellular L-ascorbic acid and has an available source of oxygen. In preferred embodiments of the fermentation culture, the microalgae can be selected from the group consisting of organisms of the genus *Prototheca* and organisms of the species *Chlorella protothecoides.* Further, preferred embodiments of the fermentation culture include a fermentation culture having a pH of less than about 6, more preferably less than about 5.5, and even more preferably less than about 5.0.

### DETAILED DESCRIPTION OF THE INVENTION

Microorganisms The microorganisms for production of L-ascorbic acid at low pH are microalgae of the genus Prototheca, especially the species Prototheca zopfii and Prototheca moriformis, and microalgae of the species Chlorella protothecoides. The method has been demonstrated with: Prototheca zopfii strain BTR 1254, Prototheca moriformis strain BTR 1385 (ATCC 75669), Chlorella protothecoides BTR 902 (ATCC 75667). Prototheca zopfii strain UTEX 1438 is capable of producing L-ascorbic acid at high pH and should also produce it at low pH.

Prototheca zopfii strain UTEX 1438 was obtained from the Culture Collection of Algae, Department of Botany, University of Texas at Austin, Austin, Texas, 78713-7640, USA. Cultures are available to the public for a nominal charge, currently $25.00 each. Prototheca zopfii strain BTR 1254, Prototheca moriformis strain BTR 1385 and Chlorella protothecoides strain BTR 902 were collected from the wild. Prototheca moriformis BTR 1385 (ATCC 75669) and Chlorella protothecoides BTR 902 (ATCC 75667) were deposited with the American Type Culture Collection (ATCC), 12301 Parklawn Drive Rockville, MD 20852, USA, on Feb. 9, 1994.

Medium The culture medium includes the carbon source, a variety of salts and, generally, trace metals. The carbon source can be any carbon source suitable for fermentation of microorganisms of the present invention. In particular, the carbon source can be selected from ethanol, glycerol and glucose and preferably is glucose. The glucose source may be glucose or any carbohydrate that can be converted in situ to glucose, e.g. molasses, corn syrup, etc.

To optimally promote, but not inhibit or unduly limit, cell growth, a non-repressing/non-limiting amount of the glucose source should be used in the fermentor. Although the optimum concentration of the glucose source may vary from organism to organism, it is readily determined by trial. Timely additions that maintain the glucose source in the 5-30 g/L range are normally sufficient to promote cell growth while avoiding glucose inhibition.

Desirably, other additives are present initially along with glucose source and may be continually added to the medium to maintain their concentrations. These include alkali metal phosphates, e.g., sodium and potassium phosphates, particularly as the dibasic sodium phosphate and the monobasic potassium phosphate. The total amount of the dibasic sodium phosphate is typically about 1-2 total g/L, preferably about 1-1.5 total g/L, and more preferably about 1.3 total g/L. The amount of dibasic sodium phosphate initially present in the fermentor is typically about 35-50%, more typically about 40-45%, of the total amount of dibasic sodium phosphate added. The total amount of monobasic potassium phosphate is usually about 1.5-3 g/L, more usually about 2-2.5 g/L. The amount initially present is generally about 40-50%, more usually about 45 to 50%, of the total amount.

A biologically acceptable chelating agent, such as trisodium citrate is advantageously added in a total amount of about 0.8-1.2 g/L, usually about 1.0 g/L. A biologically acceptable mineral acid is added to maintain the trace metals in solution and to neutralize the ammonia that is usually used as the nitrogen source. Conveniently, concentrated sulfuric acid is used at about 1-2 mL/L, typically about 1.2-1.5 mL/L.

Magnesium, about 0.1-0.2 g/L, preferably about 0.1-0.15 g/L, is added as a physiologically acceptable salt, e.g., sulfate. Since iron and copper accelerate the breakdown of extracellular L-ascorbic acid and, therefore, inhibit its accumulation in the medium, the amount of these metals used is limited. Iron (+2) is present initially at about 2-5 mg/L, preferably about 3-4 mg/L, and is not included in any subsequent additions. Copper is present in relatively minute amounts, generally 1-50 g/g of glucose. A trace metal solution (Table 3 is added in total amount of about 10-15 mL/L, typically about 12-14 mL/L. Based on glucose, the trace metal solution corresponds to 0.1 to 0.2 mL/g. For convenience, the solution shown in Table 1 is added during the fermentation. (Solution preparation is described in Table 5) Although, a number of the salts are referred to as mono- or dibasic, it should be understood that this is a matter of convenience and not necessity. Since these compounds are buffers, the extent of protonation varies with the pH of the medium. To avoid introduction of foreign microorganisms, aseptic addition is used.

**TABLE 1**

| **Medium Formula** | |
|---|---|
| Component | Concentration (Relative to glucose) |
| glucose | 1.0 |
| trisodium citrate, dihydrate | 0.0125 |
| magnesium sulfate, anhydrous | 0.0082 |
| monobasic sodium phosphate | 0.0116 |
| monobasic potassium phosphate | 0.0238 |
| dibasic sodium phosphate | 0.0121 |
| sulfuric acid 98% (w/w) | 0.0329 |
| trace metal mixture (Table 3) | 0.1675 mL/g |

Fermentation Before inoculation, the nutrient medium is brought up to the desired temperature, typically 30-40°C, preferably about 35°C. The medium is inoculated with an actively growing culture of the desired microorganism in an amount sufficient to produce, after a reasonable growth period, a high cell density. Typical initial cell densities are 0.1-0.5 g/L, more typically 0.15-0.4 g/L, based on the dry weight of the cells. The cells are grown to a cell density of at least about 5g/l, preferably about 10-80 g/L, and more preferably 40-60 g/L. This typically requires 10-40 hours, more typically 15-25 hours.

Initially about 15-30%, typically about 20-25%, of the total glucose source is added. When the glucose concentration drops, it is replenished, as needed, by adding about 20% aliquots of the glucose-salts concentrate solution (Table 1) while keeping the total glucose concentration below 30 g/L. Conventional techniques, such as the glucose oxidase enzyme test and high pressure liquid chromatography, can be used to monitor glucose concentration in the supernatant, i.e., cell-free component of the medium. A small amount of antifoaming agent may be added during the fermentation.

Ammonia is added both as a source of nitrogen and to control pH. Consequently, the amount of nitrogen in the medium depends on its acidity and buffer capacity. Ammonia is conveniently added by adding a stream of gaseous ammonia to the flow of air, or other oxygen source, that is introduced into the fermentor.

The pH of the medium can be controlled within desired limits, such as by addition of ammonia or inorganic base as needed. The pH is preferably maintained below a pH at which significant degradation of extracellular L-ascorbic acid will occur by oxidation. In this context, significant degradation refers to degradation of more than about 20% of produced L-ascorbic acid, more particularly, greater than about 10% of produced L-ascorbic acid, and more particularly, greater than about 5% of produced extracellular L-ascorbic acid. The pH of the medium is maintained below about pH 6.0, more preferably below about pH 5.5, and most preferably below about pH 5.0. By maintaining the pH of the fermentation medium within the parameters identified above, significant advantages related to the production of L-ascorbic acid are obtained. At lower pH values, degradation of produced extracellular L-ascorbic acid is reduced. Thus, higher L-ascorbic acid productivities can be obtained. It will be recognized by those skilled in the art that extracellular L-ascorbic acid is significantly easier to recover than intracellular L-ascorbic acid. In addition, extracellular production of L-ascorbic acid can allow for development of higher productivity processes because commercial processes can be developed without the need for ultrahigh intracellular L-ascorbic acid concentrations which can cause feedback inhibition of metabolic production of L-ascorbic acid.

In one embodiment of the present invention, during the initial cell growth the pH is controlled at about 3.0-6.0, preferably at about 3.5-5.0, more preferably at about 3.5-4.5. When the cell density is greater than about 10 g/L, generally after about 10-25 hours, the pH is reduced to 2.5-5.0, preferably 2.5-4.0. This may be conveniently accomplished by temporarily stopping addition of ammonia. The pH of the broth drops due to the acid produced by the cells. When the broth reaches the desired pH, addition of ammonia is resumed.

As noted above, by operation of the fermentation process of the present invention with maintaining the pH within parameters identified above, it is possible for extracellular ascorbic acid to exist in the fermentation medium without being degraded by oxidation. Moreover, it is possible for L-ascorbic acid to accumulate in the fermentation medium to attain high concentrations of extracellular ascorbic acid without significant degradation of ascorbic acid by oxidation. Thus, while the fermentation process requires the presence of oxygen, as described in more detail below, it is possible to successfully conduct the fermentation process of the present invention producing high amounts of extracellular ascorbic acid even in the presence of an available source of oxygen. It should be noted that the available source of oxygen is not limited to air or oxygen gas, but can also include other chemical species which in the fermentation environment become converted to oxygen.

Sufficient oxygen must be added to the medium during the course of the fermentation to maintain cell growth during the initial cell growth and to maintain metabolism and L-ascorbic acid formation. Oxygen is conveniently provided by agitation and aeration of the medium. Conventional methods, such as stirring or shaking, may be used to agitate and aerate the medium. Preferably the oxygen concentration in the medium is 20-100% of the saturation value (i.e., the solubility of oxygen in the medium at atmospheric pressure and about 30-40°C) although excursions to lower concentrations may occur if fermentation is not adversely affected. The oxygen concentration of the medium can be monitored by conventional methods, such as with an oxygen probe electrode. Other sources of oxygen, such as undiluted oxygen gas and oxygen gas diluted with inert gas other than nitrogen, can be used.

Fermentation is continued until the formation of L-ascorbic acid, as evidenced by the accumulation of extracellular L-ascorbic acid, essentially ceases. The total fermentation time is typically 24-120 hr.

Typically, most of the L-ascorbic acid produced in the present process is extracellular. The cells can be removed from the broth by conventional methods, such as filtration or centrifugation, and L-ascorbic acid recovered from the cell-free supernatant by conventional methods, such as, ion exchange, chromatography, extraction, crystallization, membrane separation, reverse osmosis, distillation, chemical derivatization processes, etc. The term "chemical derivatization processes" refers to processes in which produced L-ascorbic acid is reacted with another chemical species which is easier to recover and/or more stable. For example, Cayle, U.S. Patent 4,595,659, discloses isolation of L-ascorbic acid from an aqueous fermentation medium by conventional ion exchange resin absorption and elution followed by decoloration, evaporation and crystallization. Isolation of the structurally similar isoascorbic acid from fermentation broth by a continuous multi-bed extraction system of anion-exchange resin is described by K. Shimizu, Agr. Biol. Chem. 31, 346- 353 (1967).

Recovery of L-ascorbic acid from processes of the present invention can include continuous, semi-continuous or batch processes. In continuous and semi-continuous processes, concentrations of extracellular L-ascorbic acid will not be as great as in batch recovery processes because extracellular L-ascorbic acid will be removed from the fermentation vessel during the fermentation. In fact, continuous or semi-continuous processes can be conducted in which the extracellular concentration of L-ascorbic acid remains very low or close to not being detectable.

As noted above the process of the present invention produces significant amounts of extracellular L-ascorbic acid. In particular, the process produces extracellular L-ascorbic acid such that at least about 2% of total L-ascorbic acid is extracellular, more preferably at least about 10% of total L-ascorbic acid is extracellular, and most preferably at least about 20% of total L-ascorbic acid is extracellular. By use of the present invention, production of an extracellular L-ascorbic acid concentration can be achieved which is greater than about 1 mg/l, more preferably greater than about 10 mg/l, and more preferably greater than about 20 mg/l.

A further embodiment of the present invention includes a fermentation culture which includes L-ascorbic acid-producing microalgae of the genus Prototheca or the species Chlorella protothecoides and fermentation medium having a pH between 2.5 and 6.0. In this embodiment, the fermentation medium includes extracellular L-ascorbic acid and an available source of oxygen. The L-ascorbic-producing algae can be the same as those described above. Similarly, the composition of the fermentation medium, including extracellular L-ascorbic acid, can be as is broadly described above.

### INDUSTRIAL APPLICABILITY

The method produces L-ascorbic acid by fermentation in a low pH fermentation broth (2.5-6.0). At this pH, L-ascorbic acid is not readily oxidized by the air. Consequently, it is not necessary that the L-ascorbic acid produced be intracellular. The L-ascorbic acid that accumulates in the broth is not readily oxidized by the oxygen present in the broth. L-Ascorbic acid (Vitamin C) is used as a dietary supplement to prevent scurvy. L-Ascorbic acid and some of its derivatives, such as ascorbyl palmitate, have been used as antioxidants in food.

### Example 1

This example demonstrates that Prototheca are capable of producing L-ascorbic acid (L-AA) at high pH. Most of the L-ascorbic produced was intracellular.

Medium Preparation. A solution of 0.27 g monobasic potassium phos-phate and 0.23 g dibasic sodium phosphate in 600 mL distilled water was heat-sterilized in a 1 L glass fermentor equipped with means for agitating the medium and feeding to it nutrient components, an oxygen source and other agents described below. After the medium had cooled, 5 mL of a 1.9 g/L ferrous sulfate heptahydrate solution was added through a 0.2 micrometer sterile filter.

Glucose-salts Concentrate. The components listed in Table 2 were sterilized, and combined, after cooling, to a final volume of 600 mL.

**TABLE 2**

| **Glucose-Salts Concentrate** | |
|---|---|
| Amount | Compound/Component |
| | Group 1 |
| 56 g | glucose, food-grade monohydrate (anhydrous basis) in 80 mL water |

| | Group 2 |
|---|---|
| 0.7 g | trisodium citrate dihydrate |
| 0.46 g | magnesium sulfate, anhydrous |
| 0.7 mL | sulfuric acid in 10 mL water |

| | Group 3 |
|---|---|
| 0.65 g | monobasic sodium phosphate |
| 1.3 g | monobasic potassium phosphate |
| 0.68 g | dibasic sodium phosphate in 10 mL water |

| | Group 4 |
|---|---|
| 9.4 mL | Trace metal solution (See Table 3) |

**TABLE 3**

| **Trace Metal Solution** | |
|---|---|
| Component | Concentration^{a} (mg/L) |
| calcium chloride, dihydrate | 3102 |
| manganese (II) sulfate, monohydrate | 400 |
| copper (II) sulfate, monohydrate | 16 |
| cobalt (II) chloride, pentahydrate | 40 |
| boric acid | 160 |
| zinc (II) sulfate, heptahydrate | 400 |
| sodium molybdate, dihydrate | 19 |
| vanadyl sulfate, dihydrate | 20 |
| nickel (II) nitrate, hexahydrate | 8 |
| sodium selenite | 18 |

| | |
|---|---|
| ^{a}Concentration of metal. | |

Trace Metals Solution The ingredients listed in Table 3 and 20 mL of concentrated hydrochloric acid were made up to 1-L with distilled water.

Nutrient Medium. 20 mL of the glucose-salts concentrate was added to the phosphate medium in the fermentor.

Cell Growth and L-Ascorbic Acid Production. The nutrient medium was heated and maintained at 35°C, agitation was begun at 300 rpm, air was sparged into the medium at 0.1 L/min, the pH was adjusted to 6.9 with ammonia added to the airflow. The medium was inoculated with an actively growing culture to give an initial cell density of approximately 0.3 g/L dry weight.

The cells were grown to densities of about 20-50 g/L (dry basis). The pH was controlled to 6.5-7.0 by the addition of gaseous ammonia. To maintain an excess of dissolved oxygen between 20% and 90% air saturation during the course of the fermentation, agitation was begun at 450 rpm and increased to 800 rpm. Aeration was begun at 0.2 L/min and increased to 0.6 L/min. Glucose availability in the supernatant was monitored either by the glucose oxidase enzyme test or by high pressure liquid chromatography. When the glucose concentration dropped, it was replenished by adding about 20% aliquots of the glucose-salts concentrate solution while keeping the total glucose concentration below 30 g/L. When all the glucose-salts concentrate had been added and, subsequently exhausted, the culture broth was assayed for L-ascorbic acid.

The method used for determining L-ascorbic acid (L-AA) is described by Grun and Loewus, Analytical Biochemistry (1983) 130:191-198. The method is an ionexchange procedure, using a 7.8 x 300 mm organic acid analysis column, HPX-87 (Bio-Rad Laboratories, Richmond, CA). The conditions are: mobile phase, 0.013 M nitric acid; flow rate 0.8 mL/min; pressure 1500 psig (1.04 x 10⁸/dynes/cm²); detection, absorbance at 245 nm. This system discriminates between the L- and D-isomers of ascorbic acid.

For dry weight determinations of cell density, whole broth samples were removed from cultures, 5 mL was centrifuged at 4000 x g for 5 min. and the pellet was washed once with distilled water, and then washed into a tared aluminum weighing dish. Cells were dried for 8-24 hr at 60°C and for an additional hour at 105°C. Cell weight was calculated by difference. Results are given in Table 4.

| Strain | Temp (°C) | Dry Cell Weight (g/L) | Total L-AA (mg/L) |
|---|---|---|---|
| *Prototheca zopfii* UTEX 1438^{a} | 35 | 27.4 | 37.8 |

| | | | |
|---|---|---|---|
| ^{a}Values are the average of four fermentations. | | | |

### Example 2

This example illustrates production of L-ascorbic acid by Prototheca zopfii at low pH (3.5-5.0). A significant amount of L-ascorbic acid was produced in the extra-cellular medium, even with measurable dissolved oxygen in the broth. Except as indicated, the procedure of Example 1 was followed. The fermentation was run in a 14-L fermentor, configured and controlled in the same manner as the 1-L fermentor. The concentrate used is given in Table 5.

**TABLE 5**

| **Glucose-salts Concentrate** | |
|---|---|
| Amount | Compound/Component |
| | Group 1 |
| 800 g | glucose, food-grade monohydrate (anhydrous basis) in 2.1 L of water |

| | Group 2 |
|---|---|
| 15 g | trisodium citrate dihydrate |
| 6.6 g | magnesium sulfate, anhydrous |
| 10 mL | sulfuric acid in 250 mL water |

| | Group 3 |
|---|---|
| 22 g | monobasic sodium phosphate |
| 22 g | dibasic sodium phosphate in 250 mL water |

| | Group 4 |
|---|---|
| 134 mL | Trace metal solution (See Table 3) |

Medium Preparation. A solution of 3.9 g monobasic potassium phosphate and 3.3 g dibasic sodium phosphate in 7.2 L distilled water was heat-sterilized in a 14-L glass fermentor. After the medium had cooled, 27 mL of a 6.0 g/L ferrous sulfate heptahydrate solution was added through a 0.2 micrometer sterile filter.

Cell Growth and L-Ascorbic Acid Production In addition to the ingredients listed in Table 6, the medium contained 2 mg/L thiamine hydrochloride, added aseptically after the fermentor had been heat-sterilized and cooled. The temperature was 30°C. The medium was inoculated with an actively-growing culture of Prototheca zopfii strain BTR 1254 to give an initial cell density of about 0.3 g/L dry weight.

The cells were grown to a cell density of 56 g/L dry weight (growth rate, 0.20 h⁻¹). The pH was maintained at about 3.5-5.0 by addition of gaseous ammonia. Agitation was begun at 100 rpm and increased to 800 rpm. Aeration was begun at 2.0 L/min and increased to 6.0 L/min of air. Conditions and analytical results are given in Table 6.

**TABLE 6**

| Time (hr) | pH | Total Supernatant | | | |
|---|---|---|---|---|---|
| | | C. D. (g/L) | L-AA (mg/L) | L-AA (mg/L) | Comments |
| 6 | 5.0 | 0.6 | | | |
| 12 | 4.8 | 3.3 | | | 250 rpm; 200 mL^{a} |
| 21 | | | | | 350 rpm; 4.0 L/min |
| 22 | 5.0 | 15.9 | | | 400 mL |
| 24 | 3.8 | 24.0 | 34.6 | 11.2 | 6.0 L/min; 500 mL |
| 25 | 4.0 | 32.2 | 39.7 | 15.8 | 800 rpm^{b} |
| 27 | 3.9 | 48.6 | 58.6 | 27.0 | |
| 28 | 4.0 | 53.6 | 71.6 | 71.6 | dissolved oxygen |
| | | | | | measured zero |
| 29 | 4.0 | 55.8 | 73.5 | 73.5 | |

| | | | | | |
|---|---|---|---|---|---|
| ^{a} Glucose-salts concentrate | | | | | |
| ^{b} 1050 mL of glucose salts concentrate added over the next 3 hr | | | | | |

### Example 3

This example illustrates production of L-ascorbic acid by Prototheca moriformis low pH (4.0-5.0). A significant amount of L-ascorbic acid was produced in the extra-cellular medium, even with measurable dissolved oxygen in the broth. Except as indicated, the procedure of Example 2 was followed.

Cell Growth and L-Ascorbic Acid Production In addition to the ingredients listed in Table 1, the medium contained 2 mg/L thiamine hydrochloride, added aseptically after the fermentor had been heat-sterilized and cooled. The temperature was 30°C.

The medium was inoculated with an actively-growing culture of Prototheca moriformis ATCC 75669 to give an initial cell density of about 0.3 g/L dry weight. The cells were grown to a cell density of 42 g/L dry weight (growth rate 0.23 h⁻¹). For the first 22 hr the pH was maintained at about 5.0 by addition of gaseous anhydrous ammonia. Then the pH addition of ammonia was stopped. When the pH had dropped to 4.0, addition of ammonia was resumed. It was maintained at 4.0 for the remainder of the fermentation. Results are given in Table 7.

**TABLE 7**

| | | Total Supernatant | | | | |
|---|---|---|---|---|---|---|
| Time (hr) | pH | C. D. (g/L) | L-AA (mg/L) | L-AA (mg/L) | mg L-AA/g of cells | Comments |
| 11 | 5.0 | 0.9 | | | | |
| 12 | | | | | | 250 rpm |
| 17 | 4.8 | 3.9 | | | | 400 rpm; 3.0 |
| | | | | | | L/min; 200 mL^{a} |
| 20 | 5.2 | 8.2 | | | | 400 rpm |
| 21 | | | | | | 500 mL; 4 L/min |
| 22 | 5.2 | 10.8 | | | | 400 mL^{b} |
| 24 | | | | | | 800 rpm; 5 L/min |
| 26 | 4.3 | 27.7 | 105 | 109 | 3.8 | |
| 28 | 4.1 | 34.5 | 132 | 140 | 3.8 | 850 rpm |
| 30 | 4.1 | 41.9 | 150 | 162 | 3.8 | |

| | | | | | | |
|---|---|---|---|---|---|---|
| ^{a} Glucose-salts concentrate | | | | | | |
| ^{b} plus 1115 mL over the next 6 hr | | | | | | |

### Example 4

This example illustrates production of L-ascorbic acid by Chlorella protothecoides at low pH (3.5-5.0). A significant amount of L-ascorbic acid was produced in the extra-cellular medium, even with measurable dissolved oxygen in the broth. Except as indicated, the procedure of Example 3 was followed.

Cell Growth and L-Ascorbic Acid Production The medium was inoculated with an actively-growing culture of Chlorella protothecoides ATCC 75667 to give an initial cell density of about 0.3 g/L dry weight. The cells were grown to a cell density of 37 g/L dry weight (growth rate 0.16 h⁻¹). For the first 18 hr the pH was maintained at about 5.0 by addition of gaseous anhydrous ammonia. Then the pH addition of ammonia was stopped. When the pH had dropped to 3.5, addition of ammonia was resumed. The pH was maintained at 3.5 for the remainder of the fermentation. Results are given in Table 8.

**TABLE 8**

| | | Total Supernatant | | | |
|---|---|---|---|---|---|
| Time (hr) | pH | C. D. (g/L) | L-AA (mg/L) | L-AA (mg/L) | Comments |
| 5 | 5.1 | 0.6 | | | 300 rpm; 50 mL^{a} |
| 18 | 5.0 | 4.8 | | | |
| 22 | -- | -- | | | 400 rpm; 350 mL^{a} |
| 24 | 3.4 | 11.9 | 15.2 | 1.8 | 600 mL; 4.0 L/min |
| 27 | 3.4 | 17.0 | 24.5 | 11.2 | 700 rpm^{b} |
| 32 | 3.5 | 23.5 | 64.0 | 21.0 | |
| 35 | 3.5 | 36.7 | 100.4 | 52.0 | glucose depleted |

| | | | | | |
|---|---|---|---|---|---|
| ^{a} Glucose-salts concentrate | | | | | |
| ^{b} 1800 mL of glucose salts concentrate added over the next 3 hr | | | | | |

## Claims

1. A process for the production of L-ascorbic acid, the process comprising:
(a) culturing an organism selected from the group consisting of organisms of the genus *Prototheca* and organisms of the species *Chlorella protothecoides* in a fermentation medium having a pH of less than 6.0 in the presence of an available source of oxygen; and
(b) recovering L-ascorbic acid from said fermentation medium.

2. A process, as claimed in Claim 1, wherein said organism is an organism of the genus *Prototheca.*

3. A process, as claimed in Claim 1, wherein said organism is selected from the group consisting of *Prototheca moriformis* and *Prototheca zopfii*.

4. A process, as claimed in Claim 1, wherein said process is conducted until at least about 10% of L-ascorbic acid produced is extracellular.

5. A process, as claimed in Claim 1, wherein said process is conducted until at least about 20% of L-ascorbic acid produced is extracellular.

6. A process, as claimed in Claim 1, wherein extracellular L-ascorbic acid accumulates in said fermentation medium.

7. A process, as claimed in Claim 1, wherein said step of recovering comprises recovering L-ascorbic acid from extracellular fermentation medium.

8. A process, as claimed in Claim 7, wherein said step of recovering L-ascorbic acid from extracellular fermentation medium is by a process selected from the group consisting of ion exchange, chromatography, extraction, membrane separation, reverse osmosis, distillation, chemical derivatization processes and crystallization.

9. A process, as claimed in Claim 7, wherein said step of recovering further comprises the step of recovering intracellular L-ascorbic acid.

10. A process, as claimed in Claim 1, wherein said fermentation medium has a pH of less than about 5.5.

11. A process, as claimed in Claim 1, wherein said fermentation medium has a pH of less than about 5.0.

12. A process, as claimed in Claim 1, wherein the cell density of said organism is at least about 5 g/l.

13. A process, as claimed in Claim 1, wherein significant degradation of L-ascorbic acid does not occur.

14. A fermentation culture, comprising an organism selected from the group consisting of organisms of the genus *Prototheca* and organisms of the species *Chlorella protothecoides* and a fermentation medium having a pH between 2.5 and 6.0 and comprising extracellular L-ascorbic acid wherein said fermentation culture is produced by a process comprising culturing said organism in said fermentation medium in an available source of oxygen until said extracellular L-ascorbic acid is produced by said organism.

15. A fermentation culture, as claimed in Claim 14, wherein said fermentation medium comprises at least about 1 mg/l of extracellular L-ascorbic acid.

16. A process for the production of L-ascorbic acid, the process comprising:
(a) culturing an organism selected from the group consisting of organisms of the genus *Prototheca* and organisms of the species *Chlorella protothecoides* in a fermentation medium having a pH of less than 6.0 in the presence of an available source of oxygen, wherein said process is conducted until said fermentation medium has a concentration of extracellular L-ascorbic acid of greater than about 1 mg/l; and
(b) recovering extracellular L-ascorbic acid from said fermentation medium.

17. A process for the production of L-ascorbic acid, the process comprising:
(a) culturing an organism selected from the group consisting of organisms of the genus *Prototheca* and organisms of the species *Chlorella protothecoides* in a fermentation medium having a pH of less than about 5.0 in the presence of an available source of oxygen; and
(b) recovering L-ascorbic acid from said fermentation medium.

18. A process, as claimed in Claim 17, wherein said process is conducted until at least 2% of L-ascorbic acid in said fermentation medium is extracellular.

19. A process, as claimed in Claim 17, wherein the cell density of said organism is at least about 5 g/l.

20. A process, as claimed in Claim 1, wherein said process is conducted until at least 2% of L-ascorbic acid in said fermentation medium is extracellular.

## Patentansprüche

1. Verfahren zur Herstellung von L-Ascorbinsäure, wobei das Verfahren umfasst:
(a) Kultivieren eines Organismus, der gewählt ist aus der Gruppe bestehend aus Organismen der Gattung Protocheca und Organismen der Spezies Chlorella protothecoides, in einem Fermentationsmedium mit einem pH von weniger als 6,0 in Gegenwart einer verfügbaren Sauerstoffquelle; und
(b) Gewinnen von L-Ascorbinsäure aus dem genannten Fermentationsmedium.

2. Verfahren nach Anspruch 1, bei welchem der genannte Organismus ein Organismus der Gattung Prototheca ist.

3. Verfahren nach Anspruch 1, bei welchem der genannte Organismus aus der Gruppe, bestehend aus Prototheca moriformis und Prototheca zopfii, gewählt ist.

4. Verfahren nach Anspruch 1, wobei das genannte Verfahren durchgeführt wird bis wenigstens ungefähr 10% der produzierten L-Ascorbinsäure extrazellulär vorliegt.

5. Verfahren nach Anspruch 1, wobei das genannte Verfahren durchgeführt wird bis wenigstens ungefähr 20% der produzierten L-Ascorbinsäure extrazellulär vorliegt.

6. Verfahren nach Anspruch 1, bei welchem sich extrazelluläre L-Ascorbinsäure in dem genannten Fermentationsmedium anhäuft.

7. Verfahren nach Anspruch 1, bei welchem der genannte Gewinnschritt die Gewinnung der L-Ascorbinsäure aus dem extrazellulären Fermentationsmedium umfasst.

8. Verfahren nach Anspruch 7, bei welchem der genannte Schritt des Gewinnens von L-Ascorbinsäure aus dem extrazellulären Fermentationsmedium durch ein Verfahren erfolgt, das aus der Gruppe, bestehend aus Ionenauetausch, Chromatographie, Extraktion, Membranseparation, Umkehrosmose, Destillation, chemische Derivatisierungsprozesse und Kristallisation, gewählt ist.

9. Verfahren nach Anspruch 7, bei welchem der genannte Gewinnschritt ferner den Schritt des Gewinnens intrazellulärer L-Ascorbinsäure umfasst.

10. Verfahren nach Anspruch 1, bei welchem das genannte Fermentationsmedium einen pH von weniger als ungefähr 5,5 hat.

11. Verfahren nach Anspruch 1, bei welchem das genannte Fermentationsmedium einen pH von weniger als ungefähr 5,0 hat

12. Verfahren nach Anspruch 1, bei welchem die Zelldichte des genannten Organismus weniger als ungefähr 5 g/L beträgt.

13. Verfahren nach Anspruch 1, bei welchem kein signifikanter Abbau von L-Ascorbinsäure erfolgt.

14. Fermentationskultur, die einen Organismus gewählt aus der Gruppe, bestehend aus Organismen der Gattung Prototheca und Organismen des Spezies Chlorella protothecoides, und ein Fermentationsmedium mit einem pH zwischen 2,5 und 6, 0 umfasst und extrazelluläre L-Ascorbinsäure aufweist, wobei die genannte Fermentationskultur durch ein Verfahren erzeugt wird, das ein Kultivieren des genannten Organismus in dem genannten Fermentationsmedium in einer verfügbaren Sauerstoffquelle bis die genannte extrazelluläre L-Ascorbinsäure durch den genannten Organismus erzeugt wird, umfasst.

15. Fermentationskultur nach Anspruch 14, bei welcher das genannte Fermentationsmedium wenigstens ungefähr 1 mg/L der extrazellulären L-Ascorbinsäure aufweist.

16. Verfahren zur Herstellung von L-Ascorbinsäure, wobei das Verfahren umfasst:
(a) Kultivieren eines Organismus, gewählt aus der Gruppe, bestehend aus Organismen der Gattung Prototheca und Organismen der Spezies Chlorella protothecoides, in einem Fermentationsmedium mit einem pH von weniger als 6,0 in Gegenwart einer verfügbaren Sauerstoffquelle, wobei das genannte Verfahren durchgeführt wird, bis das genannte Fermentationsmedium eine Konzentration der extrazellulären L-Ascorbinsäure von größer als ungefähr 1 mg/L hat; und
(b) Gewinnen von extrazellulärer L-Ascorbinsäure aus dem genannten Fermentationsmedium.

17. Verfahren zur Herstellung von L-Ascorbinsäure, wobei das Verfahren umfasst:
(a) Kultivieren eines Organismus, gewählt aus der Gruppe, bestehend aus Organismen der Gattung Prototheca und Organismen der Spezies Chlorella protothecoides, in einem Fermentationsmedium mit einem pH von weniger als ungefähr 6,0 in Gegenwart einer verfügbaren Sauerstoffquelle; und
(b) Gewinnen von L-Ascorbinsäure aus dem genannten Fermentationsmedium.

18. Verfahren nach Anspruch 17, wobei das genannte Verfahren durchgeführt bis wenigstens 2% der L-Ascorbinsäure in dem genannten Fermentationsmedium extrazellulär vorliegt.

19. Verfahren nach Anspruch 17, bei welchem die Zelldichte des genannten Organismus weniger als ungefähr 5 g/L beträgt.

20. Verfahren nach Anspruch 1, wobei das genannte Verfahren durchgeführt wird bis wenigstens 2% der L-Ascorbinsäure in dem genannten Fermentationsmedium extrazellulär vorliegt.

## Revendications

1. Procédé de production d'acide L-ascorbique, le procédé comprenant :
(a) la mise en culture d'un organisme sélectionné dans le groupe consistant en organismes du genre *Prototheca* et en organismes de l'espèce *Chlorella protothecoides* dans un milieu de fermentation ayant un pH inférieur à 6,0 en présence d'une source d'oxygène disponible ; et
(b) la récupération d'acide L-ascorbique dans ledit milieu de fermentation.

2. Procédé selon la revendication 1, dans lequel ledit organisme est un organisme du genre *Prototheca.*

3. Procédé selon la revendication 1, dans lequel ledit organisme est sélectionné dans le groupe consistant en *Prototheca moriformis* et *Prototheca zopfii.*

4. Procédé selon la revendication 1, dans lequel ledit procédé est conduit jusqu'à ce qu'au moins environ 10 % d'acide L-ascorbique produit soit extracellulaire.

5. Procédé selon la revendication 1, dans lequel ledit procédé est conduit jusqu'à ce qu'au moins environ 20 % d'acide L-ascorbique produit soit extracellulaire.

6. Procédé selon la revendication 1, dans lequel l'acide L-ascorbique extracellulaire s'accumule dans ledit milieu de fermentation.

7. Procédé selon la revendication 1, dans lequel ladite étape de récupération comprend la récupération d'acide L-ascorbique à partir du milieu de fermentation extracellulaire.

8. Procédé selon la revendication 7, dans lequel ladite étape de récupération d'acide L-ascorbique à partir du milieu de fermentation extracellulaire s'effectue par un procédé sélectionné dans le groupe consistant en échange d'ions, chromatographie, extraction, séparation membranaire, osmose inverse, distillation, procédés de dérivation chimique et cristallisation.

9. Procédé selon la revendication 7, dans lequel ladite étape de récupération comprend, de plus, l'étape de récupération d'acide L-ascorbique intracellulaire.

10. Procédé selon la revendication 1, dans lequel ledit milieu de fermentation a un pH inférieur à environ 5,5.

11. Procédé selon la revendication 1, dans lequel ledit milieu de fermentation a un pH inférieur à environ 5,0.

12. Procédé selon la revendication 1, dans lequel la densité cellulaire dudit organisme est au moins d'environ 5 g/l.

13. Procédé selon la revendication 1, dans lequel une dégradation importante d'acide L-ascorbique ne se produit pas.

14. Culture de fermentation, comprenant un organisme sélectionné dans le groupe consistant en organismes du genre *Prototheca* et en organismes de l'espèce *Chlorella protothecoides*, et un milieu de fermentation ayant un pH compris entre 2,5 et 6,0 et comprenant de l'acide L-ascorbique extracellulaire, dans lequel ladite culture de fermentation est produite par un procédé comprenant la mise en culture dudit organisme dans ledit milieu de fermentation dans une source d'oxygène disponible, jusqu'à ce que ledit acide L-ascorbique extracellulaire soit produit par ledit organisme.

15. Culture de fermentation selon la revendication 14, dans laquelle ledit milieu de fermentation comprend au moins environ 1 mg/l d'acide L-ascorbique extracellulaire.

16. Procédé pour la production d'acide L-ascorbique, le procédé comprenant :
(a) la mise en culture d'un organisme sélectionné dans le groupe consistant en organismes du genre *Prototheca* et en organismes de l'espèce *Chlorella protothecoides* dans un milieu de fermentation ayant un pH inférieur à 6,0 en présence d'une source d'oxygène disponible, dans lequel ledit procédé est conduit jusqu'à ce que ledit milieu de fermentation ait une concentration en acide L-ascorbique extracellulaire supérieure à environ 1 mg/l ; et
(b) la récupération d'acide L-ascorbique extracellulaire à partir dudit milieu de fermentation.

17. Procédé pour la production d'acide L-ascorbique, le procédé comprenant :
(a) la mise en culture d'un organisme sélectionné dans le groupe consistant en organismes du genre *Prototheca* et en organismes de l'espèce *Chlorella protothecoides* dans un milieu de fermentation ayant un pH inférieur à environ 5,0 en présence d'une source d'oxygène disponible ; et
(b) la récupération d'acide L-ascorbique à partir dudit milieu de fermentation.

18. Procédé selon la revendication 17, dans lequel ledit procédé est conduit jusqu'à ce qu'au moins 2 % d'acide L-ascorbique dans ledit milieu de fermentation soit extracellulaire.

19. Procédé selon la revendication 17, dans lequel la densité cellulaire dudit organisme est au moins d'environ 5 g/l.

20. Procédé selon la revendication 1, dans lequel ledit procédé est conduit jusqu'à ce qu'au moins 2 % d'acide L-ascorbique dans ledit milieu de fermentation soit extracellulaire.
